(19) Europäisches Patentamt
European Patent Office
Office européen des brevets

(11) **EP 4 699 518 A1**

(12) **EUROPEAN PATENT APPLICATION**

(43) Date of publication:
**25.02.2026 Bulletin 2026/09**

(21) Application number: **24195153.2**

(22) Date of filing: **19.08.2024**

(51) International Patent Classification (IPC):
**A61B 5/00** $^{(2006.01)}$

(52) Cooperative Patent Classification (CPC):
**A61B 5/7221**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.
5656 AG Eindhoven (NL)**

(72) Inventors:
• **REIN, Michael
5656 AG Eindhoven (NL)**
• **BRAUN, Manuela
5656 AG Eindhoven (NL)**

(74) Representative: **Philips Intellectual Property &
Standards
High Tech Campus 52
5656 AG Eindhoven (NL)**

(54) **GENERATING PHYSIOLOGICAL INFORMATION**

(57) A mechanism for generating an output signal from one or more physiological signals, where the contribution of a measure set of each physiological signal to the output signals is dependent upon a respective probabilistic model for the physiological signal.

FIG. 3

EP 4 699 518 A1

**Description**

FIELD OF THE INVENTION

The present invention relates to the field of physiological monitoring of a subject, such as a medical subject.

BACKGROUND OF THE INVENTION

In the field of medicine, subject monitoring systems are becoming increasingly relied upon to provide accurate information about the condition of the subject (e.g., a patient). In a typical subject monitoring system, one or more sensor arrangements will generate physiological signals, each of which are responsive to changes in a physiological parameter of the subject.

Examples of physiological parameters that may be represented by a physiological signal include heart rate, blood pressure, body temperature, respiratory rate, blood oxygen saturation (SpO2), end-tidal CO2, intracranial pressure, glucose measurements and so on. A physiological parameter may represent a vital sign of the subject, or some other form of physiological parameter relevant for assessing the condition of the subject.

There is a strong desire for signals generated by a subject monitoring system to be accurate. However, artifacts, such as motion artifacts, can interfere with the reliability of these signals, leading to inaccurate readings. There is therefore a demand for signals that are more reliable and representative of the true condition of the (monitored) subject.

SUMMARY OF THE INVENTION

The invention is defined by the claims.

According to examples in accordance with an aspect of the invention, there is provided a computer-implemented method for generating an output signal responsive to one or more physiological signals.

The computer-implemented method comprises: for each of the one or more physiological signals, defining a respective probabilistic model that indicates, for each potential measure of the physiological signal, a probability distribution of the true value corresponding to the potential measure; receiving, as one or more measure sets, a measure set for each of the one or more physiological signals, each measure set containing one or more measures of a respective physiological signal; and for each measure set, processing the measure set using the probabilistic model of the respective physiological signal to determine an extent to which the measure set is to contribute to the output signal; and processing the one or more measure sets to generate the output signal, wherein the output signal is further responsive to the determined extent to which each measure set is to contribute to the output signal.

The present disclosure proposes an approach for determining to what extent a measure of a physiological signal should contribute to an output signal. This extent is defined using a probabilistic model that defines or indicates a probability distribution for the true value of the measure. The probabilistic function may, for instance, be usable to define a probability that the measure represents the true value of the physiological signal and/or define a probability that a threshold for the physiological signal is breached.

The proposed approaches provide a mechanism for producing an output signal that is more likely to be accurate and robust. In particular, proposed approach reduce a risk of erroneous or anomalous measures of a physiological signal from passing through to an output signal, potentially misleading or misdirecting a clinician and/or further processing system that makes use of the output signal(s).

In some examples, the one or more physiological signals comprises a plurality of physiological signals representing a same physiological parameter; for each measure set, the step of processing the measure set using the probabilistic model of the respective physiological signal comprises: processing each measure in the measure set using the probabilistic model to determine a measure of reliability for the measure set; and comparing the determined measure of reliability of the measure set to the determined measure of reliability of each other measure set to determine an extent to which each measure set is to contribute to the output signal.

This provides a mechanism for weighted combination or selection of physiological signals to produce either a combined/hybrid/fused output signal or a selected output signal (e.g., representing only a single physiological signal). This mechanism allows for flexible processing of multiple physiological signals to generate a more reliable output signal. In particular, this flexibility enables the method to adapt to different monitoring scenarios, physiological parameters and/or mechanisms for monitoring the subject. The mechanism is also able to reliably and robustly handle cases where the relative reliability of different measure sets changes over time, dynamically adjusting the output signal generation process accordingly.

In some examples, the step of processing the one or more measure sets to generate the output signal comprises combining the one or more measure sets using a combination procedure to produce, as the output signal, a fused physiological signal representing the physiological parameter; and for each measure set, the step of comparing the determined measure of reliability of the measure set to the determined measure of reliability of each other measure set comprises identifying a weighting to be applied to each measure set during the combination process.

This provides a mechanism for performing a weighting combination of different measures representing a same physiological signal. The output signal thereby represents a combination or fusion of different physiological signals, which changes or adapts over time to changing reliabilities of the physiological signals. The weighted combination technique allows for the exploitation of information from multiple sensors or measurement techniques, improving overall accuracy by facilitating the incorporation of complementary data sources. In particular, some data sources may be less sensitive to causes of noise than other data sources, which can be taken into account when producing the output data.

In some examples, for each measure set, the step of comparing the determined measure of reliability of the measure set to the determined measure of reliability of each other measure set comprises identifying which measure set is associated with the measure of reliability indicating the highest reliability; and the step of processing the one or more measure sets comprises controlling the output signal to contain only the measure set associated with the measure of reliability indicating the highest reliability.

This provides a mechanism for selecting only a single one of the physiological signals to function or function as the output signal. This may be useful for situations where using a single high-quality signal is more appropriate than combining potentially conflicting information. This approach also has a reduced processing overload, and is also able to mitigate the impact of an extremely noisy data source on the output signal.

The computer-implemented method may comprise, for each physiological signal, defining one or more thresholds wherein: for each measure set, the step of processing the measure set using the probabilistic model of the respective physiological signal comprises: for each measure of the measure set of the respective physiological signal: processing the measure using the probabilistic model to identify the probability distribution of the true value corresponding to the measure; determining, for each threshold of the respective physiological signal, a probability that the measure breaches the threshold using the identified probability distribution of the true value corresponding to the measure; determining, for each threshold, whether or not no fewer than a predetermined number of measures in the measure set breach said threshold with a probability greater than a predetermined probability, wherein the predetermined number is non-zero; and the step of processing the one or more measure sets comprises generating the output signal to indicate whether or not, for at least one measure set, more than a predetermined number of measures in the measure set breach at least one threshold with a probability greater than the predefined probability.

This provides an approach for reliably determining whether or not the measure(s) in a measure set breach a predetermined threshold. In particular, a probability that a measure has breached the threshold is used to determine whether or not the measure contributes to the output signal.

In particular, the method is able to account for measurement uncertainty by using probability distributions rather than single point values, which allows for a more nuanced evaluation of whether a threshold has truly been exceeded, rather than relying solely on raw measured values which may contain noise or artifacts.

The use of probabilistic models enables the method to adapt to different levels of measurement precision or reliability across various physiological parameters or monitoring devices. For example, a more precise measurement technique may result in narrower probability distributions, while a less reliable method may produce wider distributions. The probabilistic approach helps reduce a risk of false alarms caused by transient fluctuations or measurement errors.

The requirement for a predetermined number of measures to breach the threshold with a certain probability helps to add robustness to the detection process. This approach may filter out isolated anomalous readings while still maintaining sensitivity to persistent changes in the physiological signal.

By incorporating the probabilistic threshold breach assessment into the generation of the output signal, the system may provide more reliable and clinically relevant information to healthcare providers. This may potentially lead to more informed decision-making and improved patient care.

In some examples, the predetermined probability is no less than 0.95, preferably no less than 0.98 and preferably no less than 0.99. By setting such a high probability threshold, the system may significantly reduce the likelihood of false positive detections, i.e., helping to filter out noise, artifacts, and transient fluctuations that could otherwise trigger false alarms.

The method may further comprise generating a user-perceptible alarm responsive to the output signal indicating that, for at least one measure set, more than a predetermined number of measures in the measure set breach at least one threshold with a probability greater than the predefined probability. Alarm generation helps improve subject safety by promptly alerting healthcare providers to potentially critical changes in a patient's condition, allowing for timely intervention. The proposed alarm mechanism is particularly effective due to its basis on probabilistic threshold breaches, which may reduce false alarms while maintaining sensitivity to clinically relevant events.

In some examples, for each probabilistic model, the probability distribution defined by the probabilistic model changes responsive to changes in the value of the potential measure. By allowing the probability distribution to change based on the value of the measure, the proposed approach is able to adapt to varying levels of uncertainty across different ranges of physiological parameters. For instance, the model may account for increased variability at extreme values, or higher precision in normal ranges. This dynamic adjustment facilitates more accurate assessment of the true physiological state, reducing false alarms in regions where measurements are inherently more variable, while maintaining sensitivity in ranges where precise detection is critical.

In some examples, each physiological signal comprises a temporal sequence of measures of the physiological parameter; and for each probabilistic model, the probability distribution defined by the probabilistic model changes responsive to changes in each value of one or more other measures of the respective physiological signal that are temporally adjacent, in the temporal sequence of measures, to the potential measure.

By considering temporally adjacent measures, the probabilistic model is able to take the temporal context of a measure into account, improving the accuracy of the probability distribution for each measure. This temporal context helps to distinguish between genuine physiological changes and transient artifacts or noise, leading to more robust and reliable processing.

In some examples the one or more physiological signals comprises a plurality of physiological signals; and for each probabilistic model, each probability distribution defined by the probabilistic model changes responsive to changes in each value of one or more other measures of one or more other physiological signals that are captured alongside the physiological signal.

By allowing the probability distribution of one physiological signal to be influenced by measures of other physiological signals, the system is able to account for physiological interdependencies between different physiological parameters, leading to more accurate and context-aware assessments of the probability distribution for a measure. By way of example, changes in one parameter (e.g., heart rate) may affect the expected distribution of another (e.g., blood pressure).

In some examples, for each probabilistic model, the probability distribution defined by the probabilistic model changes responsive to a mechanism by which the respective physiological signal is generated.

This approach recognizes that different measurement techniques or devices may have distinct characteristics that affect the reliability and accuracy of the physiological signals they generate. By adapting the probability distribution based on the specific mechanism used to generate each physiological signal, the system is able to account for known strengths, limitations, and error patterns associated with different measurement methods.

In some examples, for each probabilistic model, each probability distribution defined by the probabilistic model is a normal or Gaussian distribution. In other examples, for each probabilistic model, each probability distribution defined by the probabilistic model is an asymmetric or non-Gaussian distribution.

There is also provided a computer program product comprising computer program code means which, when executed on a computing device having a processing system, cause the processing system to perform all of the steps of any herein proposed (computer-implemented) method.

There is also provided a processing system configured to generate an output signal responsive to one or more physiological signals.

The processing system is configured to: for each of the one or more physiological signals, define a respective probabilistic model that indicates, for each potential measure of the physiological signal, a probability distribution of the true value corresponding to the potential measure; receive, as one or more measure sets, a measure set for each of the one or more physiological signals, each measure set containing one or more measures of a respective physiological signal; and for each measure set, process the measure set using the probabilistic model of the respective physiological signal to determine an extent to which the measure set is to contribute to the output signal; and process the one or more measure sets to generate the output signal, wherein the output signal is further responsive to the determined extent to which each measure set is to contribute to the output signal.

The processing system may be adapted to perform the function of any herein disclosed or proposed (computer-implemented) method and vice versa.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system in which embodiments may be employed;
Fig. 2 illustrates an example probability distribution;
Fig. 3 is a flowchart illustrating a proposed method;
Fig. 4 is a flowchart illustrating a first variant method;
Fig. 5 is a flowchart illustrating a second variant method; and
Fig. 6 illustrates a system comprising a proposed processing system.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for generating an output signal from one or more physiological signals, where the contribution of a measure set of each physiological signal to the output signals is dependent upon a respective probabilistic model for the physiological signal.

In the context of the present disclosure, different physiological signals may be generated using different mechanisms. Although possible, it is not necessary that different physiological signals represent different physiological parameters.

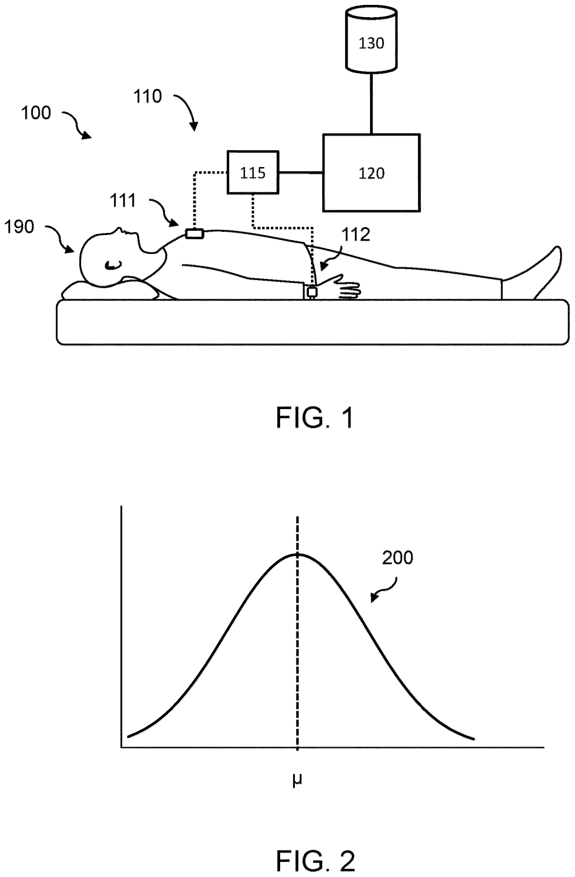

Fig. 1 illustrates a system 100 in which embodiments may be employed. The system comprises a subject monitoring system 110, a processing system 120 and a memory or database 130.

The subject monitoring system 110 is configured to generate one or more measure sets. Each measure set contains one or more measures of a respective physiological signal for a subject 190. Thus, the subject monitoring system 110 may comprise one or more subject monitoring devices 111, 112 each configured to monitor a physiological parameter of the subject, and generate a respective measure set responsive to the monitored physiological parameter. The subject monitoring system 110 may comprise a central processing system 115 for coordinating the monitoring of the physiological parameter(s) and/or receiving the physiological signal(s) from the subject monitoring devices.

Suitable examples of subject monitoring devices, and physiological parameters, are well known in the art. For instance, the subject monitoring system 110 may comprise one or more: electrocardiogram (ECG) monitors (e.g., for measuring heart rate and/or rhythm); pulse oximeters (e.g., for measuring blood oxygen saturation levels); blood pressure monitors; (e.g., for measuring systolic and/or diastolic blood pressure); thermometers (e.g., for measuring body temperature); respiratory rate monitors such as pneumography devices or acoustic sensors; glucose monitors (e.g., for measuring blood glucose levels); and so on.

The processing system 120 is configured to receive the one or more measure sets produced by the subject monitoring system 110. This may be performed by directly obtaining the measure set(s) from the subject monitoring system 110 or retrieving the measure set(s) from the memory 130.

Accordingly, the subject monitoring system 110 may be configured to store the measure set(s) in the memory 130.

The processing system 120 is configured to process the one or more measure sets to generate an output signal 195.

The output signal may, for instance, be an alarm signal configured to control the operation of an alarm (e.g., a user-perceptible output). In general, the processing system 120 may be configured to compare each measure set to one or more respective thresholds. Responsive to a threshold being breached (e.g., by each measure in the measure set or by a certain number of measures in the measure set), then the alarm signal is controlled to trigger the alarm.

As another example, the output signal may comprise a fused or processed signal. For instance, the one or more measure sets may comprise a plurality of measure sets that may be combined or fused to create the processed signal. This can be helpfully indicative of certain medical condition or pathologies that manifests or evidences itself in multiple forms of physiological signal or parameters (e.g., sepsis, stress, stroke and so on). In particular, the processed signal may represent a medical condition or pathology that is not (accurately) identifiable from a single pathological signal alone.

As yet another example, the output signal may comprise a selected one of a plurality of different measure sets. For instance, the processing system 130 may be configured to select which of a plurality of different measure sets (representing a same physiological parameter) are selected and included in the output signal.

Other suitable examples will be readily apparent to the skilled person. All examples share a same principle of one or more measure sets being processed in the generation of an output signal 195.

The present invention recognizes that the accuracy and relevance of the output signal can be improved through the use of probability models that each indicate a respective probability distribution. In particular, the invention makes use of probabilistic models to account for uncertainties and variations in physiological measurements, thereby enhancing the reliability of the generated output signal.

More specifically, the use of probabilistic models allows the proposed approach to adapt to varying levels of measurement uncertainty across different physiological signals and/or monitoring conditions. This can lead to more reliable and clinically relevant output signals, ultimately improving the quality of patient monitoring and care.

The present disclosure recognizes that the value of a measure of a physiological signal distribution may be unreliable. In particular, there is a margin of error for any value of the measure. The present disclosure recognizes that this margin of error can be granularly represented using a probability distribution, which represents the probability of potential values (e.g., surrounding a measure) to be the true value for any respective measure.

The probability distribution for any measure of a physiological signal can be defined using a probabilistic model. The probabilistic model therefore indicates, for any measure of each physiological signal, a respective probability distribution for the measure.

Fig. 2 provides an illustrative probability distribution 200 for a true value of a measure of a physiological signal.

The probability distribution represents the probability, for a particular measure of the physiological signal, of all possible values for the physiological signal representing the true value of the measure of the physiological signal.

In this scenario, the probability distribution is a Gaussian probability distribution defined by a mean $\mu$ and a standard deviation $\sigma$. The mean $\mu$ may be equal to the value of the measure of the physiological signal, but this is not essential. More complex forms of probability distributions (e.g., non-uniform probability distributions) may be definable.

Nonetheless, the skilled person will appreciate that the probability distribution for a true value of a measure of a physiological signal may be asymmetric or non-gaussian.

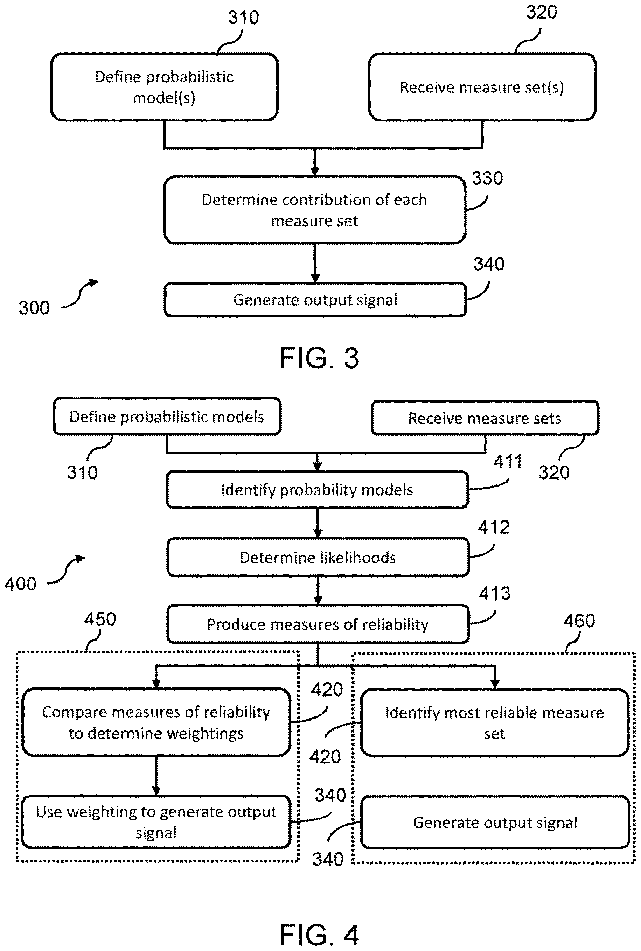

Fig. 3 is a flowchart illustrating a proposed method 300. The method 300 is configured for generating an output signal, which is responsive to one or more physiological signals.

The method 300 may be performed by a processing system, such as the processing system 110 (Fig. 1). As such, the method 300 is a computer-implemented. Method.

The method 300 comprises a process 310 of, for each of the one or more physiological signals, defining a respective probabilistic model Each probabilistic model represents or defines, for each potential measure of the physiological signal, a probability distribution of the true value corresponding to the potential measure. In other words, a probabilistic model is defined for each of the one or more physiological signals to which the generated output signal is responsive.

Step 310 may be performed, for instance, by retrieving the probabilistic model(s) from a memory or database.

The one or more measures may comprise a single measure. This may be suitable for scenarios where instantaneous or discrete measurements are taken, or where the physiological parameter being monitored does not require frequent sampling.

In other examples, the one or more measures comprise a plurality of measures (e.g., no fewer than ten measures). The use of a plurality of measures allow for potential filtering of anomalous readings or artifacts within the set.

The method 300 also comprises a step 320 of receiving, as one or more measure sets, a measure set for each of the one or more physiological signals. Each measure set contains one or more measures of a respective physiological signal. Step 320 may be performed by receiving or retrieving the measure set(s) from a subject monitoring device and/or a memory or dataset.

The method 300 also comprises a step 330 of, for each measure set, processing the measure set using the probabilistic model of the respective physiological signal to determine an extent to which the measure set is to contribute to the output signal. A number of example approaches for performing this step are described later in this disclosure, and may be dependent upon the nature of the measure set(s) and/or the purpose of the output signal.

The method 300 also comprises a step 340 of processing the one or more measure sets to generate the output signal. The output signal is further responsive to the determined extent to which each measure set is to contribute to the output signal.

Thus, the output signal is produced by processing the one or more measure sets and the determined extent to which each measure set is to contribute to the output signal. Like step 330, a number of example approaches for performing step 340 are described later in this disclosure, and may be dependent upon the nature of the measure set(s) and/or the purpose of the output signal.

Although not illustrated in Fig. 3, the method 300 may further comprise outputting the output signals, i.e., to another processing system or the like.

For example, the method may comprise sending the output signals to a user interface for display or presentation to a clinician or other healthcare professional. This could involve presenting the output signal(s), or any signal responsive to the output signal or derived therefrom, on a monitor, tablet, or other display device.

In some examples, the method may comprise storing the output signals in a memory or database, e.g., for later retrieval and analysis. This allows for tracking patient data over time, generating reports, or performing retrospective studies on the processed physiological information.

In some examples, the method may comprise transmitting the output signal(s) to one or more further processing devices. These devices may perform additional analysis, such as alarm generation, trend detection, pattern recognition, or integration with other clinical data systems. For instance, the output signals could be sent to a central monitoring station for continuous observation by medical staff, or to an electronic health record system for documentation and long-term storage.

In some examples, the output signals may be used to trigger one or more automated responses or interventions. For example, if the output signal indicates a critical or disadvantageous condition of the subject, it may automatically alert emergency response teams or activate certain medical devices to provide immediate care.

Of course, the method may also include steps (where appropriate) for formatting, adapting, modifying or otherwise processing the output signals based on the intended destination. This could involve converting data formats, applying encryption for secure transmission, or adjusting the level of detail in the output based on the recipient's needs or access privileges.

It will be appreciated that method 300 may be iteratively repeated, e.g., with a different (e.g., more up to date) one or more measure sets being obtained in each iteration of the method. This can provide real-time analysis and/or processing of measure sets.

Fig. 4 is a flowchart illustrating a method 400, which is an example of the method 300 (Fig. 3) in a first scenario.

In this first scenario, the one or more physiological signals comprises a plurality of physiological signals representing a same physiological parameter. As such, there will be a plurality of measure sets.

By way of example only, the plurality of physiological signals may comprise physiological signals representing a heart rate produced by: an electrocardiogram (ECG) monitor; a pulse oximeter using photoplethysmography (PPG); a blood pressure monitor deriving heart rate from pressure waveforms; and/or an acoustic sensor configured to detect heart sounds to estimate heart rate.

Moreover, for this first scenario and as later elucidated, the output signal represents a selection or combination of the plurality of physiological signals for the same physiological parameter.

In method 400, the step 330 comprises a sub-step 410 of processing each measure in the measure set using the probabilistic model to determine a measure of reliability for the measure set.

A variety of approaches may be employed to perform sub-step 410.

In the illustrated example, sub-step 410 comprises a sub-step 411 of processing each measure in the measure set to identify a respective probability model for the measure in the measure set. Sub-step 410 then performs a sub-step 412 of processing the measure and its respective probability distribution to determine a likelihood of each measure. The determined likelihoods is then combined, in a sub-step 413, to produce the measure of reliability for the measure set.

In this context, a likelihood may be defined as a probability (e.g., on a scale of from 0 to 1 or from 0 to 100) or a distance from a mean of the probability distribution indicated by the probabilistic model (e.g., expressed as a multiple of a standard deviation).

Approaches for performing step 411 will vary depending upon the form of the probabilistic model, as will be appreciated by the skilled person.

In one example, the probabilistic model may define the probability distribution for a plurality of different measures of the physiological signal. In this way, the probability distribution may be dependent on the value of the measure.

For instance, the probabilistic model may define at least a standard deviation (and optionally a mean) for different measures for the physiological signal, which can be used to define a normal or Gaussian distribution. This can be trivially defined in a lookup table or similar. Where the mean is not defined by the probabilistic model, the value of the measure may be defined as the mean when determining the probability distribution.

In this example, the probability distribution for a measure of the physiological signal may be defined by identifying the most proximate measure for which the probabilistic model defines a probability distribution.

Other approaches for defining a probability distribution for a measure (e.g., non-uniform or non-normal distributions) will be readily apparent to the skilled person.

In a more sophisticated example, the probabilistic model may be dependent upon the value of the measure and one or more previous and/or subsequent measures of the physiological signal (e.g., in the measure set).

For instance, the probabilistic model may be implemented using a Bayes approach. In this way, the probability distribution for a measure of a physiological signal may be at least partially dependent upon one or more earlier or historic measure(s) for the physiological signal.

By way of example, the probabilistic model may start with a prior probability distribution for a physiological measure (e.g., a default probability distribution, a user-defined probability distribution or one based on population statistics or historical data for the specific subject). As new measurements are taken of the physiological signal, the model uses Bayes' theorem to calculate a posterior probability distribution, which represents an updated belief about the true value of the physiological parameter for a particular measure.

The use of a Bayesian approach in the probabilistic model allows for a dynamic and adaptive system that can continuously refine its estimates of the true value of a measure of a physiological signal as more data of the physiological signal becomes available.

In another example, the probabilistic model may be defined using a Long Short-Term Memory (LSTM) model. LSTM models are a type of recurrent neural network architecture particularly well-suited for processing and predicting time series data, making them particularly well suited for physiological signal analysis.

In this implementation, the LSTM model may take as input a sequence of recent measures (e.g., including the measure set) of the physiological signal and output parameters defining the probability distribution for the measure being processed in step 411. For instance, the model could output the mean and standard deviation of a Gaussian distribution, or it could provide a more complex representation of the probability distribution.

The LSTM model may be trained on historical measure of the physiological signal to learn the underlying patterns and variations in the signal. Once trained, the model can be used to predict the probability distribution of the true value corresponding to each measure of the physiological signal.

In some examples, the probabilistic model may be implemented using a machine-learning method, such as a neural network. A machine-learning algorithm is any trainable algorithm that processes input data in order to produce or predict output data. Here, the input data comprises at least a measure of a measure set and the output data comprises a probability distribution.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g., $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example measures. The training output data entries correspond to a probability distribution.

The skilled person will readily appreciate how the machine-learning method (and the training thereof) can be readily adapted to receive, as input, additional features and/or input elements. For instance, the machine-learning model (forming the probabilistic model) may receive, as input any one or more of the following: the measure; historic measures; other measures of the physiological signal; other measures of other physiological signals; mechanism information (defining characteristics of the mechanism by which the measure was generated) as so on. The training dataset may be formed appropriately.

In some examples, some of which have been previously disclosed, the probability distribution defined by the probabilistic model changes responsive to changes in the value of the potential measure.

In some examples, each physiological signal comprises a temporal sequence of measures of the physiological parameter. In particular, each set of measures may comprise a temporal sequence of measures. For each probabilistic model, the probability distribution defined by the probabilistic model may changes responsive to changes in each value of one or more other measures of the respective physiological signal that are temporally adjacent, in the temporal sequence of measures, to the potential measure.

Some examples of this approach have been previously described, for instance, the Bayesian approach and/or the LTSM approach. Other examples will be readily apparent to the skilled person.

In some examples, the model may use a sliding window approach, where the probability distribution for a given measure is influenced by a fixed number of preceding and following measures in the temporal sequence.

One approach may involve the use of state-space models, such as Kalman filters or particle filters. These models can account for the temporal evolution of the physiological signal while also incorporating measurement uncertainty. In a Kalman filter implementation, for example, the model may maintain an estimate of the true physiological state and update this estimate as new measures are received, taking into account both the predicted state evolution and the uncertainty in the new measurement.

Yet another approach may employ one or more time series analysis techniques, such as autoregressive integrated moving average (ARIMA) models. These models can account for trends, seasonality, and other temporal patterns in the data, allowing for more accurate probability distributions that reflect the signal's temporal characteristics.

In some examples, the probabilistic model may incorporate multiple time scales to capture both short-term fluctuations and long-term trends in the physiological signal. For example, a hierarchical model could be used, where one level models rapid changes in the signal, while another level captures slower, more gradual changes over time.

In some examples, usable where the one or more physiological signals comprises a plurality of physiological signals, for each probabilistic model, each probability distribution defined by the probabilistic model changes responsive to changes in each value of one or more other measures of one or more other physiological signals that are captured alongside the physiological signal.

In some examples, the probabilistic model may incorporate cross-signal dependencies to account for interactions between different physiological signals. For instance, the probabilistic model may use a multivariate approach, where the probability distribution for a measure of one physiological signal is influenced not only by its own historical values but also by the values of other related physiological signals. This could be implemented using techniques such as multivariate Gaussian processes or vector autoregressive models.

In one implementation, the probabilistic model may employ a dynamic Bayesian network that represents the relationships between multiple physiological signals. The network structure may encode known physiological relationships, while the model parameters can be learned from historic or population data.

In some cases, the probabilistic model may use a hierarchical structure where there is a global model capturing overall physiological state, and individual sub-models for each physiological signal. The global model may influence the probability distributions of individual signals, allowing for a holistic representation of the subject's physiological state.

In some examples, for each probabilistic model, the probability distribution defined by the probabilistic model changes responsive to a mechanism by which the respective physiological signal is generated. As previously mentioned, different measure sets may be generated using different mechanisms or types of devices. For instance, a heartrate may be monitored by a pulse oximeter and an ECG system. This approach recognizes that different measurement techniques may have distinct error profiles, sensitivities, and vulnerabilities to artifacts.

As a crude example, each mechanism by which a respective physiological signal is generated may be associated with a different set of one or more submodels, each submodel embedding characteristics of the relevant mechanism (or device) for generating the physiological signal that influence the accuracy or reliability of measures produced by said mechanism.

For instance, the probabilistic model (or submodels) may incorporate device-specific error models or uncertainty estimates provided by the manufacturer. These could include known biases, precision limits, or characteristic error patterns associated with each measurement technique.

Other approaches, such as machine-learning models, can readily integrate mechanism information defining the mechanism by which the respective physiological signal is generated readily.

A wide variety of other approaches for producing a probability distribution for a measure from a probabilistic model will be readily apparent to the skilled person.

Approaches for performing sub-step 412 will be readily apparent to the skilled person in the art. In particular the skilled person would be readily capable of processing a value (i.e., the measure) and a probability distribution to determine a likelihood (also known as a probability) that the value is the true value.

For example, if the probability distribution is a normal (Gaussian) distribution with a mean $\mu$ and standard deviation $\sigma$, the probability P of a measure X being the true value for the physiological signal is as follows:

$$P(X) = 1 - \Phi\left(\frac{X - \mu}{\sigma}\right) \qquad (1)$$

where $\Phi$ is the cumulative distribution function of the standard normal distribution. More complex techniques, e.g., employing numerical method techniques such as Monte Carlo simulations or discrete summation of probabilities, can be employed for other forms of distributions.

Sub-step 413 may comprise an average of the likelihoods (determined in sub-step 412) to calculate the measure of reliability.

As another example, sub-step 413 may comprise generating the measure of reliability to indicate whether or not more than a predetermined number of the livelihoods (determined in sub-step 412) breach a predetermined likelihood threshold.

As another example, sub-step 413 may comprise selecting the likelihood (determined in sub-step 412) representing at least the likely measure as the measure of reliability.

As yet another example, sub-step 413 may comprise calculating the measure of reliability using a weighted average of the likelihoods of all measures in the set (which are determined from the probabilistic model). The weights may be determined based on factors such as the recency of the measure or its relative importance.

In method 400, the step 330 further comprises a sub-step 420 of comparing the determined measure of reliability of the measure set to the determined measure of reliability of each other measure set to determine an extent to which each measure set is to contribute to the output signal.

Two variants 450, 460 for performing steps 420 and 340 in the performance of method 400 are illustrated by Fig. 4. Approach configurations for previously disclosed steps will be provided where appropriate for each variant.

In a first variant 450, step 340 may comprise combining the measure sets using a combination procedure to produce, as the output signal, a fused physiological signal representing the physiological parameter. The combination procedure comprises applying a weight to each measure set during the combination procedure.

In other words, step 340 comprises performing a weighting combination of the measure sets for the same physiological parameter to produce a fused signal representing the physiological parameter. The fused signal therefore represents a combination of different measure sets.

In particular, for the first variant, step 420 comprises comparing the determined measure of reliability of the measure set to the determined measure of reliability of each other measure set to identify a weighting to be applied to each measure set during the combination process. This weighting process may be implemented in various ways to ensure that more reliable measure sets contribute more significantly to the final fused physiological signal.

In particular, the weighting process may be configured to assign higher/greater weights to measure sets with reliability measures that indicate higher reliability.

One approach to perform weighting could be to make use of linear scaling, in which the weight assigned to each measure set is directly proportional to its reliability measure. For instance, in a scenario where the reliability measure ranges from 0 (low reliability) to 1 (high reliability), if measure set A has a reliability of 0.8 and measure set B has a reliability of 0.6, their respective weights could be 0.57 (0.8 / 1.4) and 0.43 (0.6 / 1.4).

Another approach to performing weighting could be to make use of exponential weight, in which the weights may be calculated as an exponential function of the reliability measures, emphasizing differences between more and less reliable measure sets. By way of example only, in a scenario where the reliability measure ranges from 0 (low reliability) to 1 (high reliability), weights could be calculated as e'(reliability measure) and then (optionally) normalized.

Yet another approach makes use of threshold-based weighting, in which measure sets with reliability meeting certain criteria (e.g., breaching a certain threshold) may receive full weight, while those below the threshold receive reduced or zero weight. Normalization may take place where appropriate.

Once weights are determined (e.g., using any above disclosed approach), the combination procedure may involve a weighted average of the measure sets, where each measure in a set is multiplied by its corresponding weight before summing across all sets (and optionally dividing by the sum of the weights). This process ensures that the final fused physiological signal incorporates information from all available measure sets, with greater emphasis placed on the more reliable sources.

In a second variant 460, step 420 comprises identifying which measure set is associated with the measure of reliability indicating the highest reliability.

In this second variant, the step 340 of processing the one or more measure sets to generate the output signal comprises controlling the output signal to contain only the measure set associated with the measure of reliability indicating the highest reliability. Thus, only a single measure set is contained in the output signal. In this way, the output signal represents a selection of the physiological signals.

If the approach 400 is iteratively repeated as each measure set is updated, then the output signal will continually update to reflect any changes in the reliability of each measure set, providing a fused or selected output signal that is more accurate and reliable than any single one of the physiological signals by themselves.

The approach 400 may be enhanced by incorporating hysteresis control 490 to provide additional stability and reduce unnecessary fluctuations in the output signal. This can be particularly beneficial when the reliability measures of different measure sets are close to each other or when there are rapid changes in the reliability of individual measure sets.

For the first variant 450, where measure sets are combined using weights, hysteresis control 40- may involve defining a hysteresis threshold, storing previous weights, comparing new reliability measures to previous ones, applying hysteresis by recalculating weights only if the change exceeds the threshold, and updating weights gradually using a smoothing factor when recalculation is triggered.

In the second variant 460, where a single measure set is selected based on highest reliability, hysteresis control 490 may be implemented to enhance stability and reduce unnecessary fluctuations in the output signal. This approach involves several steps to ensure that the system does not switch between measure sets too frequently or in response to minor changes in reliability.

Firstly, a hysteresis margin is defined. This margin acts as a buffer, requiring a significant difference in reliability before a switch occurs.

When new reliability measures are calculated for each measure set, they are compared to the reliability measure of the currently selected set. The system only switches to a new measure set (i.e., changes which measure set is selected for the output signal) if the reliability of the new measure set exceeds the reliability of the measure current set by more than the defined hysteresis margin. This requirement ensures that minor fluctuations in reliability do not trigger unnecessary switches.

To further enhance stability, the approach may implement an additional criterion of a minimum duration for maintaining higher reliability before switching. This means that even if a new set's reliability exceeds the current set's by more than the hysteresis margin, the switch may not occur immediately. Instead, the new set must maintain this higher reliability for a specified period before the system actually switches to it.

This approach helps to prevent rapid back-and-forth switching between measure sets, which could otherwise occur if reliability measures are fluctuating around similar values. By requiring both a significant difference in reliability and potentially a sustained period of higher reliability, the system ensures that switches only occur when there is a meaningful and persistent improvement in reliability.

This approach helps avoid rapid switching between measure sets or frequent small weight adjustments, reducing instability or jitter in the output signal while still allowing adaptation to significant reliability changes.

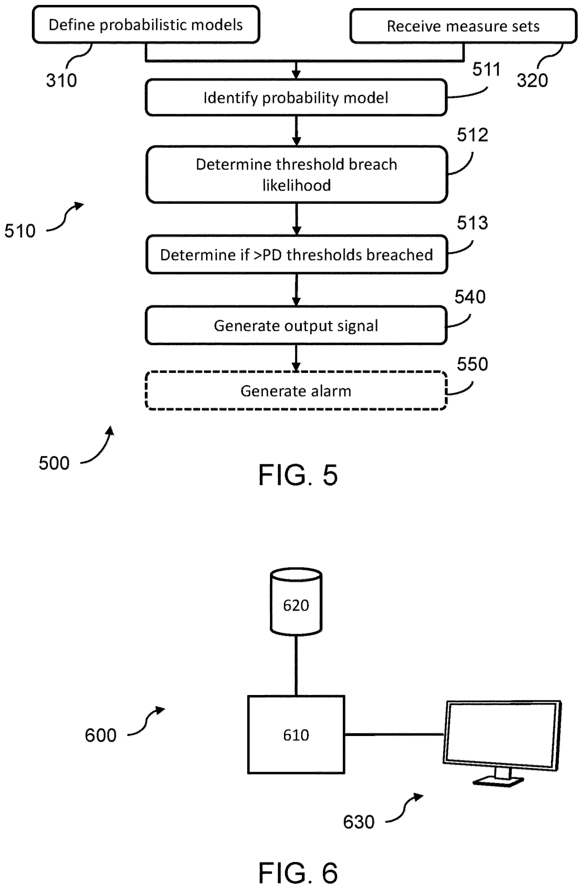

Fig. 5 is a flowchart illustrating a method 500, which is an example of the method 300 (Fig. 3) in a second scenario. In this second scenario, the output signal is designed for use as an alarm signal that indicates whether or not the measure set of a physiological signal breaches one or more (alarm) thresholds.

In method 500, step 330 (performed for each measure set) comprises performing a threshold breach test 510 for each measure and each threshold. The threshold breach test aims to identify whether the value of the measure breaches the threshold.

In this way, the threshold breach check aims to identify whether the measure contributes to the output signal (e.g., triggers an alarm) or not (e.g., does not trigger an alarm).

The threshold breach test 510 comprises a step 511 of processing the measure using the probabilistic model to identify the probability distribution of the true value corresponding to the measure.

Approaches for performing step 511 will vary depending upon the form of the probabilistic model, as will be appreciated by the skilled person.

In a crude example, the probabilistic model may simply define a default probability distribution, e.g., define a standard deviation or variance. The probability distribution for a measure can be produced by defining a Gaussian or normal distribution centered at the measure (i.e., the measure defines the mean) and using the standard deviation or variance to define the other values.

In a more complex example, the probabilistic model may define the probability distribution for a plurality of different measures of the physiological signal. In this way, the probability distribution may be dependent on the value of the measure.

For instance, the probabilistic model may define at least a standard deviation (and optionally a mean) for different measures for the physiological signal, which can be used to define a normal or Gaussian distribution. This can be trivially defined in a lookup table or similar. Where the mean is not defined by the probabilistic model, the value of the measure may be defined as the mean when determining the probability distribution.

In this example, the probability distribution for a measure of the physiological signal may be defined by identifying the most proximate measure for which a probability distribution is defined by the probabilistic model.

Other example probabilistic models and approaches for generating a probability model have been described in the context of sub-step 411, and may be readily adapted for use in performing this step.

The threshold breach test 510 also comprises a step 512 of determining, for each threshold of the respective physiological signal, a probability that the measure breaches the threshold using the identified probability distribution of the true value corresponding to the measure.

In one approach, the probability of breaching the threshold may be calculated by integrating the probability distribution function from the threshold (value) to infinity (for an upper threshold) or from negative infinity to the threshold value (for a lower threshold). This integration provides the cumulative probability of the true value of the measure exceeding or falling below the threshold, respectively.

For example, if the probability distribution is a normal (Gaussian) distribution with a mean $\mu$ and standard deviation $\sigma$, the probability P of a measure X breaching an upper threshold T may be calculated as:

$$P(X > T) = 1 - \Phi\left(\frac{T - \mu}{\sigma}\right) \qquad (1)$$

where $\Phi$ is the cumulative distribution function of the standard normal distribution.

In cases where the probability distribution is not easily integrable, e.g., for non-normal distributions, numerical methods may be employed to approximate the probability. These may include Monte Carlo simulations or discrete summation of probabilities over a fine grid of values.

The threshold breach test 510 further comprises a step 513 of, for each threshold, determining whether or not no fewer than a predetermined number (PD) of measures in the measure set breach said threshold with a probability greater than a predetermined probability, wherein the predetermined number is non-zero.

The predetermined probability may, for instance, be no less than 0.95, preferably no less than 0.98 and preferably no less than 0.99. A relatively high probability helps ensure a high level of confidence in the threshold breach detection. In particular, a high probability threshold helps to reduce a risk of false alarms while still maintaining sensitivity to clinically significant changes in the physiological signal.

In some implementations, the predetermined probability may be adjustable, e.g., dependent upon a user preference (i.e., may be user-adjustable via a user interface or the like) or based on the specific physiological parameter being monitored or the clinical context. For example, for certain critical parameters, the probability threshold may be set lower, to reduce a risk that a critical condition will be missed or overlooked.

While setting a high predetermined probability helps ensure the reliability of threshold breach detections, it should be balanced against the need for timely detection of clinically significant events. The specific values chosen may be subject to clinical validation and/or modification and may be refined based on real-world performance data and feedback from healthcare providers.

The predetermined number may be dependent upon at least the number of measures in the measure set. For instance, where the measure set comprises only a single measure, then the predetermined number will be 1. Where the measure set comprises a plurality of measures, then the predetermined number may be a nearest non-zero integer to a predetermined fraction of the total number of the plurality of measures (e.g., at least 0.3 times the number of measures in the measure set).

More particularly, the predetermined number may be set to ensure that a sufficient proportion of measures in the set indicate a threshold breach before triggering an alarm or other response. The specific value of this predetermined number may depend on various factors, such as the total number of measures in the set, the criticality of the physiological parameter being monitored, and the desired balance between sensitivity and specificity of the alarm system. This approach helps to filter out isolated anomalies or transient fluctuations that may not represent a true clinical concern.

For example, in a measure set containing 10 measures, the predetermined number might be set to 3, meaning that at least 3 out of the 10 measures must breach the threshold with a probability greater than the predetermined probability for the threshold breach to be considered significant.

The predetermined number may also be defined as a percentage of the total number of measures in the set. For instance, it could be set to 30% of the total measures, rounded up to the nearest integer. This allows the system to adapt to varying sizes of measure sets while maintaining a consistent level of sensitivity.

In some implementations, the predetermined number may be dynamically adjusted based on the current clinical context or the patient's condition. For example, for a patient in a critical care unit, the predetermined number might be set lower to increase the system's sensitivity to potential issues. Conversely, for a stable patient in a general ward, the predetermined number might be set higher to reduce false alarms.

The predetermined number may (e.g., also) be user-adjustable, e.g., via a user interface or the like.

In method 500, step 340 comprises generating the output signal to indicate whether or not, for at least one measure set, more than a predetermined number of measures in the measure set breach at least one threshold with a probability greater than the predefined probability. In this way, the output signal is responsive to the determined extent to which each measure set is to contribute to the output signal.

The method 500 may further comprise a step 550 of generating a user-perceptible alarm responsive to the output signal. The user-perceptible alarm indicates that, for at least one measure set, more than a predetermined number of measures in the measure set breach at least one threshold with a probability greater than the predefined probability.

The user-perceptible alarm may be implemented in a wide variety of forms, such as a user-audible output (e.g., a beeping sound or a voice message), a visual output (e.g., a flashing light or display on a screen), a tactile output (e.g., a vibration on a wearable device) and so on. Of course, the alarm may be a combination of multiple modalities, such as audiovisual alerts coupled with tactile feedback, to increase a likelihood that the user-perceptible alarm is noticed and acted upon promptly.

The method 500 may be configured to adjust the characteristics of the user-perceptible alarm based on the severity or persistence of the threshold breach. For example, a minor or short-duration breach may trigger a low-intensity alarm, while a severe or prolonged breach may result in a more intense or frequent alarm signal.

For instance, in a heart rate monitoring scenario, a minor breach may occur when the heart rate slightly exceeds the upper threshold for a short duration. In this case, the method may generate a low-intensity alarm, such as a soft beeping sound or a small visual indicator on a display. However, if the heart rate significantly exceeds the upper threshold or remains above the threshold for an extended period, the method may escalate the alarm to a more intense level. This could involve a louder, more frequent audible alert, a prominently flashing visual warning, and activation of a vibration feature on a wearable device.

The method may also or otherwise adjust or define characteristics of the user-perceptible alarm responsive to the probability and frequency of threshold breaches, ensuring that healthcare providers or patients are appropriately alerted to potential health risks while minimizing unnecessary disturbances for minor fluctuations.

The skilled person would be readily capable of developing a processing system for carrying out any herein described method. Thus, each step of the flow chart may represent a different action performed by a processing system, and may be performed by a respective module of the processing system.

Fig. 6 illustrates a system 600 according to a proposed embodiment, which includes a processing system 600, a (optional) memory 610 and a (optional) user interface.

The processing system 600 is configured to perform any herein disclosed method. The processing system 600 is, by itself, a proposed embodiment.

Thus, the processing system is configured to: for each of the one or more physiological signals, define a respective probabilistic model that indicates, for each potential measure of the physiological signal, a probability distribution of the true value corresponding to the potential measure; receive, as one or more measure sets, a measure set for each of the one or more physiological signals, each measure set containing one or more measures of a respective physiological signal; and for each measure set, process the measure set using the probabilistic model of the respective physiological signal to determine an extent to which the measure set is to contribute to the output signal; and process the one or more measure sets to generate the output signal, wherein the output signal is further responsive to the determined extent to which each measure set is to contribute to the output signal.

The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

The processing system 610 may be configured to receive or retrieve the one or more measures and/or the probabilistic model from the memory 610. In some examples, the processing system is configured to store the generated output signal(s) in the memory.

In some examples, the processing system 610 is configured to control the operation or function of the user interface 620 responsive to the determined output signals. For instance, the processing system 620 may be configured to control a display provided by the user interface to provide a visual representation of the output signals. As another example, where the processing system is configured to generate a user-perceptible alarm, the alarm may be generated using the user interface.

The user interface may be embodied in any suitable form, e.g., as a display screen, a speaker or other audio output device and/or a tactile output device (such as a vibrating element).

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. A computer-implemented method for generating an output signal responsive to one or more physiological signals, the computer-implemented method comprising:

   for each of the one or more physiological signals, defining a respective probabilistic model that indicates, for each potential measure of the physiological signal, a probability distribution of the true value corresponding to the potential measure;
   receiving, as one or more measure sets, a measure set for each of the one or more physiological signals, each measure set containing one or more measures of a respective physiological signal; and
   for each measure set, processing the measure set using the probabilistic model of the respective physiological signal to determine an extent to which the measure set is to contribute to the output signal; and
   processing the one or more measure sets to generate the output signal, wherein the output signal is further responsive to the determined extent to which each measure set is to contribute to the output signal.

2. The computer-implemented method of claim 1, wherein:

   the one or more physiological signals comprises a plurality of physiological signals representing a same physiological parameter;
   for each measure set, the step of processing the measure set using the probabilistic model of the respective physiological signal comprises:

      processing each measure in the measure set using the probabilistic model to determine a measure of reliability for the measure set; and
      comparing the determined measure of reliability of the measure set to the determined measure of reliability of each other measure set to determine an extent to which each measure set is to contribute to the output signal.

3. The computer-implemented method of claim 2, wherein:

   the step of processing the one or more measure sets to generate the output signal comprises combining the one or more measure sets using a combination procedure to produce, as the output signal, a fused physiological signal representing the physiological parameter; and
   for each measure set, the step of comparing the determined measure of reliability of the measure set to the determined measure of reliability of each other measure set comprises identifying a weighting to be applied to each measure set during the combination process.

4. The computer-implemented method of claim 2, wherein:

   for each measure set, the step of comparing the determined measure of reliability of the measure set to the determined measure of reliability of each other measure set comprises identifying which measure set is associated with the measure of reliability indicating the highest reliability; and
   the step of processing the one or more measure sets comprises controlling the output signal to contain only the measure set associated with the measure of reliability indicating the highest reliability.

5. The computer-implemented method of claim 1, further comprising, for each physiological signal, defining one or more thresholds wherein:

   for each measure set, the step of processing the measure set using the probabilistic model of the respective physiological signal comprises:

      for each measure of the measure set of the respective physiological signal:

processing the measure using the probabilistic model to identify the probability distribution of the true value corresponding to the measure;

determining, for each threshold of the respective physiological signal, a probability that the measure breaches the threshold using the identified probability distribution of the true value corresponding to the measure;

determining, for each threshold, whether or not no fewer than a predetermined number of measures in the measure set breach said threshold with a probability greater than a predetermined probability, wherein the predetermined number is non-zero; and

the step of processing the one or more measure sets comprises generating the output signal to indicate whether or not, for at least one measure set, more than a predetermined number of measures in the measure set breach at least one threshold with a probability greater than the predefined probability.

6. The computer-implemented method of claim 5, wherein the predetermined probability is no less than 0.98 and preferably no less than 0.99.

7. The computer-implemented method of any one of claims 5 or 6, further comprising generating a user-perceptible alarm responsive to the output signal indicating that, for at least one measure set, more than a predetermined number of measures in the measure set breach at least one threshold with a probability greater than the predefined probability.

8. The computer-implemented method of any one of claims 1 to 7, wherein, for each probabilistic model, the probability distribution defined by the probabilistic model changes responsive to changes in the value of the potential measure.

9. The computer-implemented method of any one of claims 1 to 8, wherein:

each physiological signal comprises a temporal sequence of measures of the physiological parameter; and
for each probabilistic model, the probability distribution defined by the probabilistic model changes responsive to changes in each value of one or more other measures of the respective physiological signal that are temporally adjacent, in the temporal sequence of measures, to the potential measure.

10. The computer-implemented method of any one of claims 1 to 9, wherein:

the one or more physiological signals comprises a plurality of physiological signals; and
for each probabilistic model, each probability distribution defined by the probabilistic model changes responsive to changes in each value of one or more other measures of one or more other physiological signals that are captured alongside the physiological signal.

11. The computer-implemented method of any one of claims 1 to 10, wherein, for each probabilistic model, the probability distribution defined by the probabilistic model changes responsive to a mechanism by which the respective physiological signal is generated.

12. The computer-implemented method of any one of claims 1 to 11, wherein, for each probabilistic model, each probability distribution defined by the probabilistic model is a normal or Gaussian distribution.

13. The computer-implemented method of any one of claims 1 to 11, wherein, for each probabilistic model, each probability distribution defined by the probabilistic model is an asymmetric or non-Gaussian distribution.

14. A computer program product comprising computer program code means which, when executed on a computing device having a processing system, cause the processing system to perform all of the steps of the method according to any one of claims 1 to 13.

15. A processing system configured to generate an output signal responsive to one or more physiological signals, the processing system being configured to:

for each of the one or more physiological signals, define a respective probabilistic model that indicates, for each potential measure of the physiological signal, a probability distribution of the true value corresponding to the potential measure;

receive, as one or more measure sets, a measure set for each of the one or more physiological signals, each measure set containing one or more measures of a respective physiological signal; and

for each measure set, process the measure set using the probabilistic model of the respective physiological signal to determine an extent to which the measure set is to contribute to the output signal; and

process the one or more measure sets to generate the output signal, wherein the output signal is further responsive to the determined extent to which each measure set is to contribute to the output signal.

y

y

y

FIG. 1

FIG. 2

FIG. 3

FIG. 4

18

```
┌─────────────────────────────┐          ┌─────────────────────────────┐
│  Define probabilistic models│          │     Receive measure sets    │
└─────────────────────────────┘          └─────────────────────────────┘
           310                                    511      320

                    ┌─────────────────────────────┐
                    │   Identify probability model │
                    └─────────────────────────────┘

                                                        512
                    ┌─────────────────────────────┐
                    │  Determine threshold breach  │
                    │          likelihood          │
                    └─────────────────────────────┘

                                                        513
                    ┌─────────────────────────────┐
                    │ Determine if >PD thresholds  │
                    │           breached           │
                    └─────────────────────────────┘

                                                        540
                    ┌─────────────────────────────┐
                    │     Generate output signal   │
                    └─────────────────────────────┘

                                                        550
                    ┌ ─ ─ ─ ─ ─ ─ ─ ─ ─ ─ ─ ─ ─ ─ ┐
                    │       Generate alarm         │
                    └ ─ ─ ─ ─ ─ ─ ─ ─ ─ ─ ─ ─ ─ ─ ┘
```

510

500

# FIG. 5

600

620

610

630

# FIG. 6

Europäisches
Patentamt

European
Patent Office

Office européen
des brevets

## EUROPEAN SEARCH REPORT

**Application Number**

EP 24 19 5153

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/022336 A1 (TEIXEIRA RODRIGO [US]) 26 January 2012 (2012-01-26) * paragraphs [0038], [0064], [0117], [0118], [0190], [0204], [0263] * ----- | 1-15 | INV. A61B5/00 |
| X | US 2010/274102 A1 (TEIXEIRA RODRIGO E [US]) 28 October 2010 (2010-10-28) * paragraphs [0047] - [0052], [0072] * ----- | 1-15 | |
| X | SHI HAN ET AL: "An Adaptive Multi-Homogeneous Sensor Weight Calculation Method for Body Sensor Networks", IEEE ACCESS, vol. 7, 22 August 2019 (2019-08-22), pages 121629-121644, XP011744707, DOI: 10.1109/ACCESS.2019.2936831 [retrieved on 2019-09-06] * pp. 121629-121630, 121635-121636 * ----- | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 24 January 2025 | Vanderperren, Yves |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
.......................................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 24 19 5153

This annex lists the patent family members relating to the patent documents cited in the above-mentioned European search report.
The members are as contained in the European Patent Office EDP file on
The European Patent Office is in no way liable for these particulars which are merely given for the purpose of information.

24-01-2025

| Patent document cited in search report | Publication date | Patent family member(s) | | Publication date |
|---|---|---|---|---|
| US 2012022336 A1 | 26-01-2012 | NONE | | |
| US 2010274102 A1 | 28-10-2010 | EP | 2512325 A2 | 24-10-2012 |
| | | US | 2010274102 A1 | 28-10-2010 |
| | | WO | 2010124034 A2 | 28-10-2010 |

EPO FORM P0459

For more details about this annex : see Official Journal of the European Patent Office, No. 12/82